# NEW EUROPEAN PATENT SPECIFICATION

(11) **EP 2 591 119 B2**
(45) Date of publication and mention of the opposition decision: **28.09.2022**
(45) Mention of the grant of the patent: 02.07.2014
(21) Application number: 11743899.4
(22) Date of filing: 15.07.2011
(51) Int. Cl.: C12P 7/06, C12P 7/10

(54) **Fermentation process with GH61 polypeptides**
Fermentationsverfahren mit GH61 Polypeptiden
Procédé de fermentation avec des polypeptides GH61

(30) Priority: 07.07.2010 US 362023 P
(43) Date of publication of application: 15.05.2013
(73) Proprietor: Novozymes North America, Inc., Franklinton, NC 27525 (US)
(72) Inventor: KANG, Zhengfang, Raleigh North Carolina 27614 (US)
(74) Representative: NZ EPO Representatives
(86) International application number: PCT/US2011/044122
(87) International publication number: WO 2012/006642

(56) References cited:
- WO-A-2009/135898
- HARRIS ET AL: "Stimulation of lignocellulosic biomass hydrolysis by proteins of glycoside hydrolase family 61: Structure and function of a large, enigmatic family", BIOCHEMISTRY, vol. 49, 15 March 2010 (2010-03-15), pages 3305-3316, XP002608645,
- HARA ET AL: "Cloning and sequence analysis of endoglucanase genes from an industrial fungus, Aspergillus kawachii", BIOSCIENCE, BIOTECHNOLOGY, AND BIOCHEMISTRY, vol. 67, 2003, pages 2010-2013, XP002597178,
- GAO ET AL: "Purification and characterization of a novel cellobiohydrolase (PdCel6A) from Penicillium decumbens JU-A10 for bioethanol production", BIORESOURCE TECHNOLOGY, vol. 102, 14 June 2011 (2011-06-14), pages 8339-8342, XP002661700,

## Description

### TECHNICAL FIELD

The present invention relates to processes of producing fermentation products from lignocellulose-containing material. Specifically, the invention relates to enhancing fermentation in processes of producing ethanol from lignocellulosic material using one or more GH61 polypeptides

### BACKGROUND ART

A vast number of commercial products that are difficult to produce synthetically are today produced by fermenting organisms. Such products including alcohols (e.g., ethanol, methanol, butanol, 1,3-propanediol); organic acids (e.g., citric acid, acetic acid, itaconic acid, lactic acid, gluconic acid, gluconate, lactic acid, succinic acid, 2,5-diketo-D-gluconic acid); ketones (e.g., acetone); amino acids (e.g., glutamic acid); gases (e.g., H₂ and CO₂), and more complex compounds, including, for example, antibiotics (e.g., penicillin and tetracycline); enzymes; vitamins (e.g., riboflavin, B₁₂, beta-carotene); and hormones. Fermentation is also commonly used in the consumable alcohol (e.g., beer and wine), dairy (e.g., in the production of yogurt and cheese), leather, and tobacco industries.

A vast number of processes of producing fermentation products, such as ethanol, by fermentation of sugars provided by degradation of starch-containing and/or lignocellulose-containing material are known in the art.

However, production of fermentation products, such as ethanol, from such plant materials is still too costly. Therefore, there is a need for providing processes that can shorten the fermentation time, increase the rate of fermentation, or increase the yield of the fermentation product and thereby reduce the production costs.

### SUMMARY OF THE INVENTION

In the first aspect the invention relates to processes of producing fermentation products from lignocellulose-containing material, comprising the steps of:
(a) pre-treating lignocellulose-containing material;
(b) hydrolyzing the material of step (a);
(c) fermenting with a fermenting organism wherein one or more GH61 polypeptides are added after hydrolysis is complete,
wherein the hydrolysis and fermentation steps are carried out as separate hydrolysis and fermentation, and the hydrolysis is taken to completion before initiation of fermentation.

The invention also relates to the use of GH61 polypeptides in fermentation processes to increase the rate of fermentation during fermentation, wherein one or more GH61 polypeptides are added after hydrolysis is complete and the hydrolysis and fermentation steps are carried out as separate hydrolysis and fermentation, and the hydrolysis is taken to completion before initiation of fermentation.

### BRIEF DESCRIPTION OF THE FIGURES

Fig. 1 demonstrates the effect of GH61a polypeptide on ethanol yield during fermentation over time.
Fig. 2 demonstrates the effect of GH61a polypeptide on glucose concentration during fermentation over time
Fig. 3 demonstrates the effect of GH61 a polypeptide on xylose concentration during fermentation over time.

### DETAILED DESCRIPTION OF THE INVENTION

### Family 61 glycoside hydrolase (GH61)

According to the invention GH61 polypeptides are polypeptides that fall into the glycoside hydrolase Family 61 according to Henrissat B., 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696.

WO 2005/074647, WO 2005/074656, and WO 2010/138754 disclose GH61 polypeptides from *Thielavia terrestris, Thermoascus aurantiacus,* and *Aspergillus fumigatus,* respectively. Such polypeptides are characterized by their ability to enhance cellulolytic activity. The term "cellulolytic enhancing activity" means a biological activity catalyzed by a GH61 polypeptide that enhances the hydrolysis of a cellulosic material by enzyme having cellulolytic activity.

Cellulolytic enhancing activity is determined by measuring the increase in reducing sugars or the increase of the total of cellobiose and glucose from the hydrolysis of a cellulosic material by cellulolytic enzyme under the following conditions: 1-50 mg of total protein/g of cellulose in PCS (Pretreated Corn Stover), wherein total protein is comprised of 50-99.5% w/w cellulolytic enzyme protein and 0.5-50% w/w protein of a GH61 polypeptide having cellulolytic enhancing activity for 1-7 days at 50°C compared to a control hydrolysis with equal total protein loading without cellulolytic enhancing activity (1-50 mg of cellulolytic protein/g of cellulose in PCS).

The GH61 polypeptides having cellulolytic enhancing activity enhance the hydrolysis of a cellulosic material catalyzed by enzyme having cellulolytic activity by reducing the amount of cellulolytic enzyme required to reach the same degree of hydrolysis.

Recent progress concerning the use of GH61 is disclosed in Biochemistry 49: 3305-3316, March 2010, and Bioresource Technology 102: 8339-8342, June 2011.

Surprisingly, the inventors have found that GH61 polypeptides are capable of enhancing fermentation in the production of fermentation products from fermentable sugars derived from plant material using a fermenting organism. The plant material is lignocellulose-containing material.

The invention relates to a process of producing a fermentation product from lignocellulose-containing material, comprising the steps of:
(a) pre-treating lignocellulose-containing material;
(b) hydrolyzing the material of step (a);
(c) fermenting with a fermenting organism wherein one or more GH61 polypeptides are added after hydrolysis is complete,
wherein the hydrolysis and fermentation steps are carried out as separate hydrolysis and fermentation, and the hydrolysis is taken to completion before initiation of fermentation.

In an embodiment the GH61 polypeptide is dosed at a concentration of 0.01-10 mg-protein/g-TS, preferably 0.1-5 mg-protein/ g TS.

### Fermenting Organism

The phrase "fermenting organism" refers to any organism, including bacterial and fungal organisms, suitable for producing a desired fermentation product. The fermenting organism may be C6 and/or C5 fermenting organisms, or a combination thereof. Both C6 and/or C5 fermenting organisms are well known in the art.

Suitable fermenting organisms are able to ferment, *i.e*., convert, fermentable sugars, such as glucose, fructose, maltose, xylose, mannose, galactose and or arabinose, directly or indirectly into the desired fermentation product.

Examples of fermenting organisms include fungal organisms such as yeast. Preferred yeast includes strains of the genus *Saccharomyces,* in particular strains of *Saccharomyces cerevisiae* or *Saccharomyces uvarum;* a strain of *Pichia,* preferably *Pichia stipitis* such as *Pichia stipitis* CBS 5773 or *Pichia pastoris;* a strain of the genus *Candida,* in particular a strain of *Candida utilis, Candida arabinofermentans, Candida diddensii, Candida sonorensis, Candida shehatae, Candida tropicalis,* or *Candida boidinii.* Other fermenting organisms include strains of *Hansenula,* in particular *Hansenula polymorpha* or *Hansenula anomala; Kluyveromyces,* in particular *Kluyveromyces fragilis* or *Kluyveromyces marxianus;* and *Schizosaccharomyces,* in particular *Schizosaccharomyces pombe.*

Preferred bacterial fermenting organisms include strains of *Escherichia,* in particular *Escherichia coli,* strains of *Zymomonas,* in particular *Zymomonas mobilis,* strains of *Zymobacter,* in particular *Zymobactor palmae,* strains of *Klebsiella* in particular *Klebsiella oxytoca,* strains of *Leuconostoc,* in particular *Leuconostoc mesenteroides,* strains of *Clostridium,* in particular *Clostridium butyricum,* strains of *Enterobacter,* in particular *Enterobacteraerogenes* and strains of *Thermoanaerobacter,* in particular *Thermoanaerobacter* BG1L1 (Appl. Microbiol. Biotech. 77: 61-86) and *Thermoanarobacter ethanolicus, Thermoanaerobacter thermosaccharolyticum,* or *Thermoanaerobacter mathranii.* Strains of *Lactobacillus* are also envisioned as are strains of *Corynebacterium glutamicum R, Bacillus thermoglucosidaisus,* and *Geobacillus thermoglucosidasius.*

The fermenting organism may be a C6 sugar fermenting organism, such as a strain of, e.g., *Saccharomyces cerevisiae.*

In connection with fermentation of lignocellulose derived materials, C5 sugar fermenting organisms are contemplated. Most C5 sugar fermenting organisms also ferment C6 sugars. Examples of C5 sugar fermenting organisms include strains of *Pichia,* such as of the species *Pichia stipitis.* C5 sugar fermenting bacteria are also known. Also some *Saccharomyces cerevisae* strains ferment C5 (and C6) sugars. Examples are genetically modified strains of *Saccharomyces spp.* that are capable of fermenting C5 sugars include the ones concerned in, e.g., Ho et al., 1998, Applied and Environmental Microbiology 64: 1852-1859 and Karhumaa et al., 2006, Microbial Cell Factories 5:18, and Kuyper et al., 2005, FEMS Yeast Research 5: 925-934. The fermenting organism may be a fungal cell transformed with a xylose isomerase gene as disclose in WO 2003/062430 (Nedalco) or WO 2010/074577 (Nedalco).

Certain fermenting organisms' fermentative performance may be inhibited by the presence of inhibitors in the fermentation media and thus reduce ethanol production capacity. Compounds in biomass hydrozylates and high concentrations of ethanol are known to inhibit the fermentative capacity of certain yeast cells. Pre-adaptation or adaptation methods may reduce this inhibitory effect. Typically pre-adaptation or adaptation of yeast cells involves sequentially growing yeast cells, prior to fermentation, to increase the fermentative performance of the yeast and increase ethanol production. Methods of yeast pre-adaptation and adaptation are known in the art. Such methods may include, for example, growing the yeast cells in the presence of crude biomass hydrolyzates; growing yeast cells in the presence of inhibitors such as phenolic compounds, furaldehydes and organic acids; growing yeast cells in the presence of non-inhibiting amounts of ethanol; and supplementing the yeast cultures with acetaldehyde. The fermenting organism may be a yeast strain subjected to one or more pre-adaptation or adaptation methods prior to fermentation.

In one embodiment the fermenting organism is added to the fermentation medium so that the viable fermenting organism, such as yeast, count per mL of fermentation medium is in the range from 10⁵ to 10¹², preferably from 10⁷ to 10¹⁰, especially about 5×10⁷.

Commercially available yeast includes, *e.g.,* RED STAR^{™} and ETHANOL RED^{™} yeast (available from Fermentis/Lesaffre, USA), FALI (available from Fleischmann's Yeast, USA), SUPERSTART^{™} and THERMOSACC^{™} fresh yeast (available from Ethanol Technology, WI, USA), BIOFERM AFT and XR (available from NABC - North American Bioproducts Corporation, GA, USA), GERT STRAND (available from Gert Strand AB, Sweden), and FERMIOL^{™} (available from DSM Specialties).

According to the invention the fermenting organism capable of producing a desired fermentation product from fermentable sugars, such as, e.g., glucose, fructose maltose, xylose, galactose and/or arabinose, is preferably grown under precise conditions at a particular growth rate. When the fermenting organism is introduced into/added to the fermentation medium the inoculated fermenting organism pass through a number of stages. Initially growth does not occur. This period is referred to as the "lag phase" and may be considered a period of adaptation. During the next phase referred to as the "exponential phase" the growth rate gradually increases. After a period of maximum growth the rate ceases and the fermenting organism enters "stationary phase". After a further period of time the fermenting organism enters the "death phase" where the number of viable cells declines.

In one embodiment the GH61 polypeptide is added to the fermentation medium when the fermenting organism is in lag phase.

In one embodiment the GH61 polypeptide is added to the fermentation medium when the fermenting organism is in exponential phase.

In one embodiment the GH61 polypeptide is added to the fermentation medium when the fermenting organism is in stationary phase.

### Fermentation Products

The term "fermentation product" means a product produced by a process including fermenting using a fermenting organism. Fermentation products contemplated according to the invention include alcohols (e.g., ethanol, methanol, butanol); organic acids (e.g., citric acid, acetic acid, itaconic acid, lactic acid, succinic acid, gluconic acid); ketones (e.g., acetone); amino acids (e.g., glutamic acid); gases (e.g., H₂ and CO₂); antibiotics (e.g., penicillin and tetracycline); enzymes; vitamins (e.g., riboflavin, B₁₂, beta-carotene); and hormones. The fermentation product may be ethanol, e.g., fuel ethanol; drinking ethanol, i.e., potable neutral spirits; or industrial ethanol or products used in the consumable alcohol industry (e.g., beer and wine), dairy industry (e.g., fermented dairy products), leather industry and tobacco industry. Preferred beer types comprise ales, stouts, porters, lagers, bitters, malt liquors, happoushu, high-alcohol beer, low-alcohol beer, low-calorie beer or light beer. Preferred fermentation processes used include alcohol fermentation processes. The fermentation product, such as ethanol, obtained according to the invention, may preferably be used as fuel. However, in the case of ethanol it may also be used as potable ethanol.

### Fermentation Medium

The phrase "fermentation media" or "fermentation medium" refers to the environment in which fermentation is carried out and comprises the fermentation substrate, that is, the carbohydrate source that is metabolized by the fermenting organism(s), and may include the fermenting organism(s).

The fermentation medium may comprise nutrients and growth stimulator(s) for the fermenting organism(s). Nutrient and growth stimulators are widely used in the art of fermentation and include nitrogen sources, such as ammonia; vitamins and minerals, or combinations thereof.

Following fermentation, the fermentation media or fermentation medium may further comprise the fermentation product.

### Fermentation

The starting material used in processes of the invention is lignocellulose-containing material. The fermentation conditions are determined based on, e.g., the kind of material, the available fermentable sugars, the fermenting organism(s) and/or the desired fermentation product. One skilled in the art can easily determine suitable fermentation conditions. The fermentation may according to the invention be carried out at conventionally used conditions. Preferred fermentation processes are anaerobic processes.

The processes of the invention may be performed as a batch, fed batch or as a continuous process. Fermentations may be conducted in an ultrafiltration system where the retentate is held under recirculation in the presence of solids, water, and the fermenting organism, and where the permeate is the desired fermentation product containing liquid. Equally contemplated areprocesses conducted in continuous membrane reactors with ultrafiltration membranes and where the retentate is held under recirculation in presence of solids, water, the fermenting organism(s) and where the permeate is the fermentation product containing liquid.

After fermentation the fermenting organism may be separated from the fermented slurry and recycled.

Fermentations are conventionally carried out using yeast, such as *Saccharomyces cerevisae,* as the fermenting organism. However, bacteria and filamentous fungi may also be used as fermenting organisms. Some bacteria have higher fermentation temperature optimum than, *e.g., Saccharomyces cerevisae.* Therefore, fermentations may in such cases be carried out at temperatures as high as 75°C, e.g., between 40-70°C, such as between 50-60°C. However, bacteria with a significantly lower temperature optimum down to around room temperature (around 20°C) are also known. Examples of suitable fermenting organisms can be found in the "Fermenting Organisms" section above.

For ethanol production using yeast, the fermentation may go on for 24 to 96 hours, in particular for 35 to 60 hours. The fermentation may be carried out at a temperature between 20 to 40°C, preferably 26 to 34°C, in particular around 32°C. The pH may be from pH 3 to 8, preferably around pH 5 to 6.

Other fermentation products may be fermented at temperatures known to the skilled person in the art to be suitable for the fermenting organism in question.

### Fermentation of Lignocellulose-Derived Sugars

As mentioned above different kinds of fermenting organisms may be used for fermenting sugars derived from lignocellulose-containing materials. Fermentations are typically carried out by yeast, bacteria or filamentous fungi, including the ones mentioned in the "Fermenting Organisms" section above. If the aim is C6 fermentable sugars the conditions are usually similar to starch fermentations. However, if the aim is to ferment C5 sugars (e.g., xylose) or a combination of C6 and C5 fermentable sugars the fermenting organism(s) and/or fermentation conditions may differ.

Bacteria fermentations may be carried out at higher temperatures, such as up to 75°C, e.g., between 40-70°C, such as between 50-60°C, than conventional yeast fermentations, which are typically carried out at temperatures from 20-40°C. However, bacteria fermentations at temperature as low as 20°C are also known. Fermentations are typically carried out at a pH in the range between 3 and 7, preferably from pH 3.5 to 6, such as around pH 5. Fermentations are typically ongoing for 24-96 hours.

### Recovery

Subsequent to fermentation the fermentation product may be separated from the fermentation medium. The fermentation medium may be distilled to extract the desired fermentation product or the desired fermentation product may be extracted from the fermentation medium by micro or membrane filtration techniques. Alternatively, the fermentation product may be recovered by stripping. Methods for recovery are well known in the art.

### Production of Fermentation Products from Lignocellulose-Containing Material

In this aspect, the invention relates to processes of producing fermentation products from lignocellulose-containing material. Conversion of lignocellulose-containing material into fermentation products, such as ethanol, has the advantages of the ready availability of large amounts of feedstock, including wood, agricultural residues, herbaceous crops, municipal solid wastes etc. Lignocellulose-containing materials typically primarily consist of cellulose, hemicellulose, and lignin and are often referred to as "biomass".

The structure of lignocellulose is not directly accessible to enzymatic hydrolysis. Therefore, the lignocellulose-containing material has to be pre-treated, e.g., by acid hydrolysis under adequate conditions of pressure and temperature, in order to break the lignin seal and disrupt the crystalline structure of cellulose. This causes solubilization of the hemicellulose and cellulose fractions. The cellulose and hemicelluloses can then be hydrolyzed enzymatically, e.g., by cellulolytic and/or hemicellulolytic enzymes, to convert the carbohydrate polymers into fermentable sugars which may be fermented into desired fermentation products, such as ethanol. Optionally the fermentation product may be recovered, e.g., by distillation as also described above.

### SSF, HHF and SHF

Hydrolysis and fermentation may be carried out as a simultaneous hydrolysis and fermentation step (SSF). In general this means that combined/simultaneous hydrolysis and fermentation are carried out at conditions (e.g., temperature and/or pH) suitable, preferably optimal, for the fermenting organism(s) in question. The hydrolysis step and fermentation step may be carried out as hybrid hydrolysis and fermentation (HHF). HHF typically begins with a separate partial hydrolysis step and ends with a simultaneous hydrolysis and fermentation step. The separate partial hydrolysis step is an enzymatic cellulose saccharification step typically carried out at conditions (e.g., at higher temperatures) suitable, preferably optimal, for the hydrolyzing enzyme(s) in question. The subsequent simultaneous hydrolysis and fermentation step is typically carried out at conditions suitable for the fermenting organism(s) (often at lower temperatures than the separate hydrolysis step).

According to the invention, the hydrolysis and fermentation steps are carried out as separate hydrolysis and fermentation, where the hydrolysis is taken to completion before initiation of fermentation. This is often referred to as "SHF". According to the invention the GH61 polypeptides to enhance fermentation are added after hydrolysis is complete. In a further embodiment, the solid and liquid phases of the hydrolysate are separated before the GH61 polypeptide is added to the liquid phase (i.e the fermentation medium). In this embodiment, the GH61 polypeptide can be added before, during or after the fermenting organism is added to the fermentation medium.

### Pre-treatment

The lignocellulose-containing material may according to the invention be pre-treated before being hydrolyzed and fermented. The pre-treated material may be hydrolyzed, preferably enzymatically, before fermentation. The goal of pre-treatment is to separate and/or release cellulose, hemicellulose and/or lignin and this way improve the rate of enzymatic hydrolysis.

According to the invention pre-treatment step (a) may be a conventional pre-treatment step known in the art. Pre-treatment may take place in aqueous slurry. The lignocellulose-containing material may during pre-treatment be present in an amount between 10-80 wt. %, preferably between 20-50 wt. %.

### Chemical, Mechanical and/or Biological Pre-treatment

The lignocellulose-containing material may according to the invention be chemically, mechanically and/or biologically pre-treated before hydrolysis and/or fermentation. Mechanical treatment (often referred to as physical pre-treatment) may be used alone or in combination with subsequent or simultaneous hydrolysis, especially enzymatic hydrolysis, to promote the separation and/or release of cellulose, hemicellulose and/or lignin.

Preferably, the chemical, mechanical and/or biological pre-treatment is carried out prior to the hydrolysis and/or fermentation. Alternatively, the chemical, mechanical and/or biological pre-treatment is carried out simultaneously with hydrolysis, such as simultaneously with addition of one or more cellulolytic enzymes, or other enzyme activities mentioned below, to release fermentable sugars, such as glucose and/or maltose.

The pre-treated lignocellulose-containing material may be washed and/or detoxified before or after hydrolysis step (b). This may improve the fermentability of, e.g., dilute-acid hydrolyzed lignocellulose-containing material, such as corn stover. Detoxification may be carried out in any suitable way, e.g., by steam stripping, evaporation, ion exchange, resin or charcoal treatment of the liquid fraction or by washing the pre-treated material. Other detoxification methods are described in WO 2008/076738 or WO 2008/134254 from Novozymes.

### Chemical Pre-treatment

The pre-treatment may be chemical pretreatment. "Chemical pre-treatment" refers to any chemical treatment which promotes the separation and/or release of cellulose, hemicellulose and/or lignin. Examples of suitable chemical pre-treatment steps include treatment with; for example, dilute acid, lime, alkaline, organic solvent, ammonia, sulphur dioxide, carbon dioxide. Further, wet oxidation and pH-controlled hydrothermolysis are also contemplated chemical pre-treatments. The pre-treatment may be acid pretreatment. Preferably, the chemical pre-treatment is acid treatment, more preferably, a continuous dilute acid treatment and/or mild acid treatment, such as, treatment with sulfuric acid, or another organic acid, such as acetic acid, citric acid, tartaric acid, succinic acid, or mixtures thereof. Other acids may also be used. Mild acid treatment means in the context of the present invention that the treatment pH lies in the range from 1-5, preferably from pH 1-3. The acid concentration may be in the range from 0.1 to 2.0 wt % acid, preferably sulphuric acid. The acid may be mixed or contacted with the material to be fermented and the mixture may be held at a temperature in the range of 160-220°C, such as 165-195°C, for periods ranging from minutes to seconds, e.g., 1-60 minutes, such as 2-30 minutes or 3-12 minutes. Addition of strong acids, such as sulphuric acid, may be applied to remove hemicellulose. This enhances the digestibility of cellulose. In dilute acid pretreatment, the lignocellulse-containing material may be mixed with dilute acid, typically H₂SO₄, and water to form a slurry, heated by steam to the desired temperature, and after a residence time flashed to atmospheric pressure. The dilute acid pretreatment can be performed with a number of reactor designs, e.g., plug-flow reactors, counter-current reactors, or continuous counter-current shrinking bed reactors (Duff and Murray, 1996, supra; Schell et al., 2004, Bioresource Technol. 91: 179-188; Lee et al., 1999, Adv. Biochem. Eng. Biotechnol. 65: 93-115).

Cellulose solvent treatment, also contemplated according to the invention, has been shown to convert about 90% of cellulose to glucose. It has also been shown that enzymatic hydrolysis could be greatly enhanced when the lignocellulosic structure is disrupted. Alkaline H₂O₂, ozone, organosolv (uses Lewis acids, FeCl₃, (Al)₂SO₄ in aqueous alcohols), glycerol, dioxane, phenol, or ethylene glycol are among solvents known to disrupt cellulose structure and promote hydrolysis (Mosier et al., 2005, Bioresource Technology 96: 673-686).

Alkaline chemical pre-treatment with base, e.g., NaOH, Na₂CO₃ and/or ammonia or the like, is also within the scope of the inventive process. Therefore, the pre-treatment may be alkaline pretreatment. Lime pretreatment is performed with calcium carbonate, sodium hydroxide, or ammonia at low temperatures of 85-150°C and residence times from 1 hour to several days (Wyman et al., 2005, Bioresource Technol. 96: 1959-1966; Mosier et al., 2005, Bioresource Technol. 96: 673-686). Pre-treatment methods using ammonia are described in, e.g., WO 2006/110891, WO 2006/110899, WO 2006/110900, WO 2006/110901. The pre-treatment may be ammonia pretreatment.

Wet oxidation techniques involve use of oxidizing agents, such as: sulphite based oxidizing agents or the like. Wet oxidation is a thermal pretreatment performed typically at 180-200°C for 5-15 minutes with addition of an oxidative agent such as hydrogen peroxide or overpressure of oxygen (Schmidt and Thomsen, 1998, Bioresource Techno/. 64: 139-151; Palonen et al., 2004, Appl. Biochem. Biotechnol. 117: 1-17; Varga et al., 2004, Biotechnol. Bioeng. 88: 567-574; Martin et al., 2006, J. Chem. Technol. Biotechnol. 81: 1669-1677). The pretreatment is performed at preferably 1-40% dry matter, more preferably 2-30% dry matter, and most preferably 5-20% dry matter, and often the initial pH is increased by the addition of alkali such as sodium carbonate.

A modification of the wet oxidation pretreatment method, known as wet explosion (combination of wet oxidation and steam explosion), can handle dry matter up to 30%. In wet explosion, the oxidizing agent is introduced during pretreatment after a certain residence time. The pretreatment is then ended by flashing to atmospheric pressure (WO 2006/032282).

Another example of solvent pre-treatments includes treatment with DMSO (Dimethyl Sulfoxide) or the like. Other examples of suitable pretreatment methods are described by Schell et al., 2003, Appl. Biochem. and Biotechnol. 105-108: 69-85, and Mosier et al., 2005, Bioresource Technology 96: 673-686, and U.S. Published Application 2002/0164730.

Chemical pre-treatment is generally carried out for 1 to 60 minutes, such as from 5 to 30 minutes, but may be carried out for shorter or longer periods of time dependent on the material to be pre-treated.

Other examples of suitable pre-treatment methods are described by Schell et al., 2003, Appl. Biochem and Biotechn. 105-108: 69-85, and Mosier et al., 2005, Bioresource Technology 96: 673-686, and US publication no. 2002/0164730.

### Mechanical Pre-treatment

The pre-treatment may be mechanical or physical pretreatment. As used in context of the present invention the term "mechanical pre-treatment" refers to any mechanical or physical pre-treatment which promotes the separation and/or release of cellulose, hemicellulose and/or lignin from lignocellulose-containing material. For example, mechanical pre-treatment includes various types of milling, irradiation, steaming/steam explosion, and hydrothermolysis.

Mechanical pre-treatment includes comminution (mechanical reduction of the particle size). Comminution includes dry milling, wet milling and vibratory ball milling. Mechanical pre-treatment may involve high pressure and/or high temperature (steam explosion). High pressure means pressure in the range from 300 to 600 psi, preferably 400 to 500 psi, such as around 450 psi. High temperature means temperatures in the range from about 100 to 300°C, preferably from about 140 to 235°C. Mechanical pre-treatment may be a batch-process, steam gun hydrolyzer system which uses high pressure and high temperature as defined above. A Sunds Hydrolyzer (available from Sunds Defibrator AB (Sweden) may be used for this.

### Combined Chemical and Mechanical Pre-treatment

Both chemical and mechanical pre-treatments may be carried out involving, for example, both dilute or mild acid pretreatment and high temperature and pressure treatment. The chemical and mechanical pretreatment may be carried out sequentially or simultaneously, as desired.

Accordingly, the lignocellulose-containing material may be subjected to both chemical and mechanical pre-treatment to promote the separation and/or release of cellulose, hemicellulose and/or lignin.

The pre-treatment may be carried out as a dilute and/or mild acid steam explosion step. Pre-treatment may be carried out as an ammonia fiber explosion step (or AFEX pretreatment step), or ammonia percolation (APR).

Pretreatment may be carried out as an ammonia fiber explosion step (AFEX pretreatment step). Ammonia fiber explosion (AFEX) involves treating cellulosic material with liquid or gaseous ammonia at moderate temperatures such as 90-100°C and high pressure such as 17-20 bar for 5-10 minutes, where the dry matter content can be as high as 60% (Gollapalli et al., 2002, Appl. Biochem. Biotechnol. 98: 23-35; Chundawat et al., 2007, Biotechnol. Bioeng. 96: 219-231; Alizadeh et al., 2005, Appl. Biochem. Biotechnol. 121: 1133-1141; Teymouri et al., 2005, Bioresource Technol. 96: 2014-2018). AFEX pretreatment results in the depolymerization of cellulose and partial hydrolysis of hemicellulose. Lignin-carbohydrate complexes are cleaved.

### Biological Pre-treatment

The pre-treatment may be biological pretreatment.

As used in the present invention the term "biological pre-treatment" refers to any biological pre-treatment which promotes the separation and/or release of cellulose, hemicellulose, and/or lignin from the lignocellulose-containing material. Biological pre-treatment techniques can involve applying lignin-solubilizing microorganisms (see, for example, Hsu, T.-A., 1996, Pretreatment of biomass, in Handbook on Bioethanol: Production and Utilization, Wyman, C. E., ed., Taylor & Francis, Washington, DC, 179-212; Ghosh, P., and Singh, A., 1993, Physicochemical and biological treatments for enzymatic/microbial conversion of lignocellulosic biomass, Adv. Appl. Microbiol. 39: 295-333; McMillan, J. D., 1994, Pretreating lignocellulosic biomass: a review, in Enzymatic Conversion of Biomass for Fuels Production, Himmel, M. E., Baker, J. O., and Overend, R. P., eds., ACS Symposium Series 566, American Chemical Society, Washington, DC, chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson, L., and Hahn-Hagerdal, B., 1996, Fermentation of lignocellulosic hydrolysates for ethanol production, Enz. Microb. Tech. 18: 312-331; and Vallander, L., and Eriksson, K.-E. L., 1990, Production of ethanol from lignocellulosic materials: State of the art, Adv. Biochem. Eng./Biotechnol. 42: 63-95).

### Hydrolysis

Before fermentation the pre-treated lignocellulose-containing material may be hydrolyzed in order to break the lignin seal and disrupt the crystalline structure of cellulose. Hydrolysis may be carried out enzymatically. According to the invention the pre-treated lignocellulose-containing material to be fermented may be hydrolyzed by one or more hydrolases (class E.C. 3 according to Enzyme Nomenclature), preferably one or more carbohydrases including cellulolytic enzymes and hemicellulolytic enzymes, or combinations thereof. Further, protease, alpha-amylase, glucoamylase and/or the like may also be present during hydrolysis and/or fermentation as the lignocellulose-containing material may include some, e.g., starchy and/or proteinaceous material.

The enzyme(s) used for hydrolysis may be capable of directly or indirectly converting carbohydrate polymers into fermentable sugars, such as glucose and/or maltose, which can be fermented into a desired fermentation product, such as ethanol.

The carbohydrase(s) has(have) cellulolytic enzyme activity and/or hemicellulolytic enzyme activity.

Hydrolysis is carried out using a cellulolytic enzyme preparation further comprising one or more polypeptides having cellulolytic enhancing activity. In an embodiment the polypeptides with cellulolytic enhancing activity is of the family GH61. In a preferred embodiment the polypeptide(s) having cellulolytic enhancing activity is(are) of family GH61A origin. Examples of suitable and preferred cellulolytic enzyme preparations and polypeptides having cellulolytic enhancing activity are described in the "Cellulolytic Enzymes" section and "Cellulolytic Enhancing Polypeptides" sections below.

In an embodiment of the process of the invention the GH61 polypeptide is a GH61A polypeptide. The GH61A polypeptide may be derived from *Thermoascus aurantiacus,* preferably the GH61A polypeptide described in WO 2005/074656 as SEQ ID NO: 1 or SEQ ID NO: 2.

The GH61 polypeptide may be a GH61 E polypeptide. The GH61 E polypeptide may be derived from *Thielavia terrestris,* preferably the GH61 E polypeptide described in WO 2005/074647 as SEQ ID NO: 7 or SEQ ID NO: 8.

The GH61 polypeptide may be a GH61 B polypeptide. The GH61 B polypeptide may be derived from *Aspergillus fumigates,* preferably the GH61 B described in WO 2010/138754 as SEQ ID NO: 1 or SEQ ID NO: 2.

Suitable enzymes are described in the "Enzymes" section below.

Hemicellulose polymers can be broken down by hemicellullolytic enzymes and/or acid hydrolysis to release its five and six carbon sugar components. The six carbon sugars (hexoses), such as glucose, galactose, arabinose, and mannose, can readily be fermented to fermentation products such as ethanol, acetone, butanol, glycerol, citric acid, fumaric acid etc. by suitable fermenting organisms including yeast.

Yeast is the preferred fermenting organism for ethanol fermentation. Preferred are strains of *Saccharomyces,* especially strains of the species *Saccharomyces cerevisiae,* preferably strains which are resistant towards high levels of ethanol, *i.e.,* up to, e.g., about 10, 12, 15 or 20 vol. % or more ethanol.

Enzymatic hydrolysis is preferably carried out in a suitable aqueous environment under conditions which can readily be determined by one skilled in the art. Hydrolysis may be carried out at suitable, preferably optimal, conditions for the enzyme(s) in question.

Suitable process time, temperature and pH conditions can readily be determined by one skilled in the art. Preferably, hydrolysis is carried out at a temperature between 25 and 70°C, preferably between 40 and 60°C, especially around 50°C. The step is preferably carried out at a pH in the range from 3-8, preferably pH 4-6. Hydrolysis is typically carried out for between 12 and 96 hours, preferable 16 to 72 hours, more preferably between 24 and 48 hours.

Fermentation of lignocellulose derived material is carried out as described above.

### Lignocellulose-Containing Material (Biomass)

Any suitable lignocellulose-containing material is contemplated in context of the present invention. Lignocellulose-containing material may be any material containing lignocellulose. The lignocellulose-containing material contains at least 50 wt. %, preferably at least 70 wt. %, more preferably at least 90 wt. % lignocellulose. It is to be understood that the lignocellulose-containing material may also comprise other constituents such as cellulosic material, such as cellulose, hemicellulose and may also comprise constituents such as sugars, such as fermentable sugars and/or un-fermentable sugars.

Lignocellulose-containing material is generally found, for example, in the stems, leaves, hulls, husks, and cobs of plants or leaves, branches, and wood of trees. Lignocellulosic material can also be, but is not limited to, herbaceous material, agricultural residues, forestry residues, municipal solid wastes, waste paper, and pulp and paper mill residues. It is understood herein that lignocellulose-containing material may be in the form of plant cell wall material containing lignin, cellulose, and hemi-cellulose in a mixed matrix.

The lignocellulose-containing material may be selected from one or more of corn fiber, rice straw, pine wood, wood chips, poplar, bagasse, and paper and pulp processing waste.

Other examples of suitable lignocellulose-containing material include corn stover, corn cobs, hard wood such as poplar and birch, soft wood, cereal straw such as wheat straw, switch grass, Miscanthus, rice hulls, municipal solid waste (MSW), industrial organic waste, office paper, or mixtures thereof.

The lignocellulose-containing material may be corn stover or corn cobs. The lignocellulose-containing material may be corn fiber. The lignocellulose-containing material may be switch grass. The lignocellulose-containing material may be bagasse.

### Enzymes

Even if not specifically mentioned in context of a process of the invention, it is to be understood that one or more enzymes used in any process of the invention is used in an "effective amount."

### Cellulolytic Activity

The phrase "cellulolytic activity" as used herein are understood as comprising enzymes having cellobiohydrolase activity (EC 3.2.1.91), e.g., cellobiohydrolase I and cellobiohydrolase II, as well as endo-glucanase activity (EC 3.2.1.4) and beta-glucosidase activity (EC 3.2.1.21).

At least three categories of enzymes are important for converting cellulose into fermentable sugars: endo-glucanases (EC 3.2.1.4) that cut the cellulose chains at random; cellobiohydrolases (EC 3.2.1.91) which cleave cellobiosyl units from the cellulose chain ends and beta-glucosidases (EC 3.2.1.21) that convert cellobiose and soluble cellodextrins into glucose. Among these three categories of enzymes involved in the biodegradation of cellulose, cellobiohydrolases seems to be the key enzymes for degrading native crystalline cellulose.

The cellulolytic activity may be in the form of a preparation of enzymes of fungal origin, such as from a strain of the genus *Trichoderma,* preferably a strain of *Trichoderma reesei;* a strain of the genus *Humicola,* such as a strain of *Humicola insolens;* or a strain of *Chrysosporium,* preferably a strain of *Chrysosporium lucknowense.*

The cellulolytic enzyme preparation may contain one or more of the following activities: cellulase, hemicellulase, cellulolytic enzyme enhancing activity, beta-glucosidase activity, endoglucanase, cellubiohydrolase, or xylose isomerase.

The cellulase may be a composition as defined in PCT/US2008/065417 published as WO 2008/151079. Specifically, the cellulase composition may be the one used in Example 1 (Cellulase preparation 2) described below. The cellulolytic enzyme preparation comprising a polypeptide having cellulolytic enhancing activity, is preferably a family GH61A polypeptide, preferably the one disclosed in WO 2005/074656 (Novozymes). The cellulolytic enzyme preparation may further comprise a beta-glucosidase, such as a beta-glucosidase derived from a strain of the genus *Trichoderma,* such as *Trichoderma reesei, Aspergillus,* such as *Aspergillus fumigatus* (WO 2005/047499) or *Aspergillus oryzae* (WO 2002/095014), or *Penicillium,* such as *Penicillium brasilianum,* including the *Aspergillus oryzae* beta-glucosidase fusion protein having beta-glucosidase activity disclosed in WO 2008/057637.

The cellulolytic enzyme preparation may also comprises a CBH II enzyme, preferably *Thielavia terrestris* cellobiohydrolase II CEL6A. The cellulolytic enzyme preparation may also comprise cellulolytic enzymes, preferably one derived from *Trichoderma reesei, Humicola insolens* or *Chrysosporium lucknowense.*

The cellulolytic enzyme preparation may also comprising a polypeptide having cellulolytic enhancing activity (GH61A) disclosed in WO 2005/074656; a beta-glucosidase (fusion protein disclosed in WO 2008/057637) and cellulolytic enzymes derived from *Trichoderma reesei.*

The cellulolytic preparation may comprise *Trichoderma reesei* cellulases, *Thermoascus aurantiacus* GH61A polypeptide having cellulolytic enhancing activity (WO 2005/074656), *Aspergillus oryzae* beta-glucosidase fusion protein (WO 2008/057637), and *Aspergillus aculeatus* xylanase (Xyl II in WO 94/21785).

The cellulolytic preparation may comprise *Trichoderma reesei* cellulases, *Thermoascus aurantiacus* GH61A polypeptide having cellulolytic enhancing activity (WO 2005/074656), *Aspergillus fumigatus* Family 3A beta-glucosidase (WO 2005/047499), and *Aspergillus aculeatus* xylanase (Xyl II in WO 94/21785).

The cellulolytic enzyme may be the commercially available product CELLUCLAST^{®} 1.5L, CELLUZYME^{™}, Cellic^{™} CTec, or Cellic^{™} CTec2 available from Novozymes A/S, Denmark, or ACCELERASE^{™} 1000, ACCELERASE^{™} 1500 or ACCELERASE^{™} DUET (from Danisco USA Inc., USA) and FIBERZYME^{™} from Dyadic Inc, USA.

A cellulolytic enzyme may be added for hydrolyzing the pre-treated lignocellulose-containing material. The cellulolytic enzyme may be dosed in the range from 0.1-100 FPU per gram total solids (TS), preferably 0.5-50 FPU per gram TS, especially 1-20 FPU per gram TS. At least 0.1 mg cellulolytic enzyme per gram total solids (TS), preferably at least 3 mg cellulolytic enzyme per gram TS, such as between 5 and 10 mg cellulolytic enzyme(s) per gram TS may be used for hydrolysis.

### Endoglucanase (EG)

The term "endoglucanase" means an endo-1,4-(1,3;1,4)-beta-D-glucan 4-glucanohydrolase (E.C. No. 3.2.1.4), which catalyses endo-hydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity may be determined using carboxymethyl cellulose (CMC) hydrolysis according to the procedure of Ghose, 1987, Pure and Appl. Chem. 59: 257-268.

Endoglucanases may be derived from a strain of the genus *Trichoderma,* preferably a strain of *Trichoderma reesei*; a strain of the genus *Humicola,* such as a strain of *Humicola insolens;* or a strain of *Chrysosporium,* preferably a strain of *Chrysosporium lucknowense.*

### Cellobiohydrolase (CBH)

The term "cellobiohydrolase" means a 1,4-beta-D-glucan cellobiohydrolase (E.C. 3.2.1.91), which catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain.

Examples of cellobiohydroloses are mentioned above including CBH I and CBH II from *Trichoderma reseei*; *Humicola insolens* and CBH II from *Thielavia terrestris* cellobiohydrolase (CEL6A).

Cellobiohydrolase activity may be determined according to the procedures described by Lever et al., 1972, Anal. Biochem. 47: 273-279; van Tilbeurgh et al., 1982, FEBS Letters 149: 152-156; and van Tilbeurgh and Claeyssens, 1985, FEBS Letters 187: 283-288. The Lever *et al.* method is suitable for assessing hydrolysis of cellulose in corn stover and the method of van Tilbeurgh *et al.* is suitable for determining the cellobiohydrolase activity on a fluorescent disaccharide derivative.

### Beta-glucosidase

One or more beta-glucosidases may be present during hydrolysis.

The term "beta-glucosidase" means a beta-D-glucoside glucohydrolase (E.C. 3.2.1.21), which catalyzes the hydrolysis of terminal non-reducing beta-D-glucose residues with the release of beta-D-glucose. For purposes of the present invention, beta-glucosidase activity is determined according to the basic procedure described by Venturi et al., 2002, J. Basic Microbiol. 42: 55-66, except different conditions were employed as described herein. One unit of beta-glucosidase activity is defined as 1.0 µmole of p-nitrophenol produced per minute at 50°C, pH 5 from 4 mM p-nitrophenyl-beta-D-glucopyranoside as substrate in 100 mM sodium citrate, 0.01 % TWEEN^{®} 20.

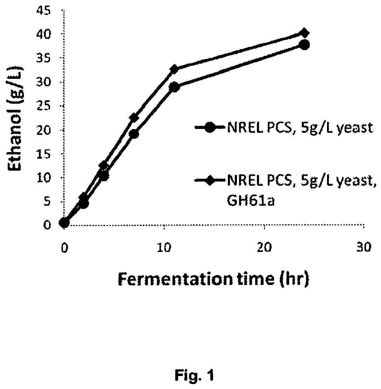

The beta-glucosidase may be of fungal origin, such as a strain of the genus *Trichoderma, Aspergillus* or *Penicillium.* The beta-glucosidase may be derived from *Trichoderma reesei,* such as the beta-glucosidase encoded by the *bgl1* gene (see Fig. 1 of EP 562003). The beta-glucosidase may be derived from *Aspergillus oryzae* (recombinantly produced in *Aspergillus oryzae* according to WO 2002/095014). The beta-glucosidase may be derived from *Aspergillus fumigatus* (recombinantly produced in *Aspergillus oryzae* according to WO 2005/047499) or *Aspergillus niger* (1981, J. Appl. 3: 157-163). The beta-glucosidase may be derived from *Penicillium brassilianum* disclosed in WO 2009/111706.

### Hemicellulase

Hemicellulose can be broken down by hemicellulases and/or acid hydrolysis to release its five and six carbon sugar components.

The lignocellulose derived material may be treated with one or more hemicellulase.

Any hemicellulase suitable for use in hydrolyzing hemicellulose, preferably into xylose, may be used. Preferred hemicellulases include xylanases, arabinofuranosidases, acetyl xylan esterase, feruloyl esterase, glucuronidases, endo-galactanase, mannases, endo or exo arabinases, exo-galactanses, and mixtures of two or more thereof. Preferably, the hemicellulase for use in the present invention is an exo-acting hemicellulase, and more preferably, the hemicellulase is an exo-acting hemicellulase which has the ability to hydrolyze hemicellulose under acidic conditions of below pH 7, preferably pH 3-7. An example of hemicellulase suitable for use in the present invention includes VISCOZYME^{™} (available from Novozymes A/S, Denmark).

The hemicellulase may be a xylanase. The xylanase may preferably be of microbial origin, such as of fungal origin (*e.g., Trichoderma, Meripilus, Humicola, Aspergillus, Fusarium*) or from a bacterium (*e.g., Bacillus).* The xylanase may be derived from a filamentous fungus, preferably derived from a strain of *Aspergillus,* such as *Aspergillus aculeatus;* or a strain of *Humicola,* preferably *Humicola lanuginosa.* The xylanase may preferably be an endo-1,4-beta-xylanase, more preferably an endo-1,4-beta-xylanase of GH10 or GH11. Examples of commercial xylanases include SHEARZYME^{™}, Cellic^{™}HTec, Cellic^{™}HTec2 and BIOFEED WHEAT^{™} from Novozymes A/S, Denmark.

The hemicellulase may be added in an amount effective to hydrolyze hemicellulose, such as, in amounts from about 0.001 to 0.5 wt. % of total solids (TS), more preferably from about 0.05 to 0.5 wt. % of TS.

Xylanases may be added in amounts of 0.001-1.0 g/kg DM (dry matter) substrate, preferably in the amounts of 0.005-0.5 g/kg DM substrate, and most preferably from 0.05-0.10 g/kg DM substrate.

### Xylose Isomerase

Xylose isomerases (D-xylose ketoisomerase) (E.C. 5.3.1.5.) are enzymes that catalyze the reversible isomerization reaction of D-xylose to D-xylulose. Some xylose isomerases also convert the reversible isomerization of D-glucose to D-fructose. Therefore, xylose isomarase is sometimes referred to as "glucose isomerase."

A xylose isomerase used in a process of the invention may be any enzyme having xylose isomerase activity and may be derived from any sources, preferably bacterial or fungal origin, such as filamentous fungi or yeast. Examples of bacterial xylose isomerases include the ones belonging to the genera *Streptomyces, Actinoplanes, Bacillus* and *Flavobacterium,* and *Thermotoga,* including *T. neapolitana* (Vieille et al., 1995, Appl. Environ. Microbiol. 61(5): 1867-1875) and *T. maritime.*

Examples of fungal xylose isomerases are derived species of *Basidiomycetes.*

A preferred xylose isomerase is derived from a strain of yeast genus *Candida,* preferably a strain of *Candida boidinii,* especially the *Candida boidinii* xylose isomerase disclosed by, *e.g.,* Vongsuvanlert et al., 1988, Agric. Biol. Chem. 52(7): 1817-1824. The xylose isomerase may preferably be derived from a strain of *Candida boidinii (Kloeckera 2201*), deposited as DSM 70034 and ATCC 48180, disclosed in Ogata et al., Agric. Biol. Chem. 33: 1519-1520 or Vongsuvanlert et al., 1988, Agric. Biol. Chem. 52(2): 1519-1520.

The xylose isomerase may be derived from a strain of *Streptomyces, e.g.,* derived from a strain of *Streptomyces murinus* (U.S. Patent No. 4,687,742); S. *flavovirens, S. albus, S. achromogenus, S. echinatus, S. wedmorensis* all disclosed in U.S. Patent No. 3,616,221. Other xylose isomerases are disclosed in U.S. Patent No. 3,622,463, U.S. Patent No. 4,351,903, U.S. Patent No. 4,137,126, U.S. Patent No. 3,625,828, HU patent no. 12,415, DE patent 2,417,642, JP patent no. 69,28,473, and WO 2004/044129.

The xylose isomerase may be either in immobilized or liquid form. Liquid form is preferred.

Examples of commercially available xylose isomerases include SWEETZYME^{™} T from Novozymes A/S, Denmark.

The xylose isomerase is added to provide an activity level in the range from 0.01-100 IGIU per gram total solids.

### Cellulolytic Enhancing Activity

As mentioned above, the polypeptides having cellulolytic enhancing activity enhance the hydrolysis of a lignocellulose derived material catalyzed by proteins having cellulolytic activity by reducing the amount of cellulolytic enzyme required to reach the same degree of hydrolysis. Such enhancement is preferably at least 0.1-fold, more preferably at least 0.2-fold, more preferably at least 0.3-fold, more preferably at least 0.4-fold, more preferably at least 0.5-fold, more preferably at least 1-fold, more preferably at least 3-fold, more preferably at least 4-fold, more preferably at least 5-fold, more preferably at least 10-fold, more preferably at least 20-fold, even more preferably at least 30-fold, most preferably at least 50-fold, and even most preferably at least 100-fold.

The hydrolysis and/or fermentation may be carried out in the presence of a cellulolytic enzyme in combination with a polypeptide having enhancing activity.

The polypeptide with cellulolytic enhancing activity is a GH61 polypeptide.

The polypeptide having enhancing activity may be a family GH61A polypeptide. WO 2005/074656 discloses an isolated polypeptide having cellulolytic enhancing activity and a polynucleotide thereof from *Thermoascus aurantiacus.* The GH61A polypeptide may be the one described in WO 2005/074656 as SEQ ID NO:1 or SEQ ID NO: 2.

The GH61 polypeptide may be a GH61E polypeptide. WO 2005/074647 discloses isolated polypeptides having cellulolytic enhancing activity and polynucleotides thereof from *Thielavia terrestris.* The GH61 E polypeptide may be the one described in WO 2005/074647 as SEQ ID NO: 7 or SEQ ID NO: 8.

The GH61 polypeptide may be a GH61 B polypeptide. The GH61 B polypeptide may be derived from *Aspergillus fumigates,* preferably the GH61 B described in WO 2010/138754 as SEQ ID NO: 1 or SEQ ID NO: 2.

U.S. Published Application No. 2007/0077630 discloses an isolated polypeptide having cellulolytic enhancing activity and a polynucleotide thereof from *Trichoderma reesei.*

Suitable examples of polypeptides having cellulolytic enhancing activity includes the ones disclosed in any of the following publications: WO 2005/074647, WO 2008/148131, WO 2009/085935, WO 2009/085859, WO 2009/085864, WO 2009/085868, WO 2010/065830, WO 2010/138754, WO 2011/005867, WO 2011/039319, WO 2011/041397, WO 2011/035027, and WO 2011/041504.

### Proteases

A protease may be added during hydrolysis in step b), fermentation in step c). The protease may be added to deflocculate the fermenting organism, especially yeast, during fermentation. The protease may be any protease. The protease may be an acid protease of microbial origin, preferably of fungal or bacterial origin. An acid fungal protease is preferred, but also other proteases can be used.

Suitable proteases include microbial proteases, such as fungal and bacterial proteases. Preferred proteases are acidic proteases, *i.e.,* proteases characterized by the ability to hydrolyze proteins under acidic conditions below pH 7.

Contemplated acid fungal proteases include fungal proteases derived from *Aspergillus, Mucor, Rhizopus, Candida, Coriolus, Endothia, Enthomophtra, Irpex, Penicillium, Sclerotium* and *Torulopsis.* Especially contemplated are proteases derived from *Aspergillus niger* (see, e.g., Koaze et al., 1964, Agr. Biol. Chem. Japan 28: 216), *Aspergillus saitoi* (see, e.g., Yoshida, 1954, J. Agr. Chem. Soc. Japan 28: 66), *Aspergillus awamori* (Hayashida et al., 1977, Agric. Biol. Chem. 42(5), 927-933, *Aspergillus aculeatus* (WO 95/02044), or *Aspergillus oryzae,* such as the pepA protease; and acidic proteases from *Mucor pusillus* or *Mucor miehei.*

Contemplated are also neutral or alkaline proteases, such as a protease derived from a strain of *Bacillus.* A particular protease contemplated for the invention is derived from *Bacillus amyloliquefaciens* and has the sequence obtainable at Swissprot as Accession No. P06832. Also contemplated are the proteases having at least 90% identity to amino acid sequence obtainable at Swissprot as Accession No. P06832 such as at least 92%, at least 95%, at least 96%, at least 97%, at least 98%, or particularly at least 99% identity.

Further contemplated are the proteases having at least 90% identity to amino acid sequence disclosed as SEQ ID NO: 1 in WO 2003/048353 such as at 92%, at least 95%, at least 96%, at least 97%, at least 98%, or particularly at least 99% identity.

Also contemplated are papain-like proteases such as proteases within E.C. 3.4.22.* (cysteine protease), such as EC 3.4.22.2 (papain), EC 3.4.22.6 (chymopapain), EC 3.4.22.7 (asclepain), EC 3.4.22.14 (actinidain), EC 3.4.22.15 (cathepsin L), EC 3.4.22.25 (glycyl endopeptidase) and EC 3.4.22.30 (caricain).

The protease may be a protease preparation derived from a strain of *Aspergillus,* such as *Aspergillus oryzae.* In another embodiment the protease is derived from a strain of *Rhizomucor,* preferably *Rhizomucor meihei.* The protease may be a protease preparation, preferably a mixture of a proteolytic preparation derived from a strain of *Aspergillus,* such as *Aspergillus oryzae,* and a protease derived from a strain of *Rhizomucor,* preferably *Rhizomucor meihei.*

Aspartic acid proteases are described in, for example, Hand-book of Proteolytic Enzymes, Edited by A.J. Barrett, N.D. Rawlings and J.F. Woessner, Aca-demic Press, San Diego, 1998, Chapter 270). Suitable examples of aspartic acid protease include, e.g., those disclosed in Berka et al., 1990, Gene 96:313; Berka et al., 1993, Gene 125: 195-198; and Gomi et al., 1993, Biosci. Biotech. Biochem. 57: 1095-1100.

Commercially available products include ALCALASE^{®}, ESPERASE^{™}, FLAVOURZYME^{™}, PROMIX^{™}, NEU-TRASE^{®}, RENNILASE^{®}, NOVOZYM^{™} FM 2.0L, and NOVOZYM^{™} 50006 (available from Novozymes A/S, Denmark) and GC106^{™} and SPEZYME^{™} FAN from Genencor Int., Inc., USA.

The protease may be present in an amount of 0.0001-1 mg enzyme protein per g DS, preferably 0.001 to 0.1 mg enzyme protein per g DS. Alternatively, the protease may be present in an amount of 0.0001 to 1 LAPU/g DS, preferably 0.001 to 0.1 LAPU/g DS and/or 0.0001 to 1 mAU-RH/g DS, preferably 0.001 to 0.1 mAU-RH/g DS.

### Use

In this aspect the invention relates to the use of GH61 polypeptides in a fermentation process wherein said polypeptides are added after hydrolysis is complete, wherein the hydrolysis and fermentation steps are carried out as separate hydrolysis and fermentation, and the hydrolysis is taken to completion before initiation of fermentation. In an embodiment GH61 polypeptides are used for improving the fermentation product yield. In an embodiment GH61 polypeptides are used for increasing the rate of fermentation during fermentation.

### MATERIALS & METHODS

### Materials:

GH61A from *Thermoascus aurantiacus* as described in WO 2005/074656 as SEQ ID NO: 1 and SEQ ID NO: 2.

GH61E from *Thielavia terrestris* as described in WO 2005/074647 as SEQ ID NO: 7 and SEQ ID NO: 8.

GH61B from *Aspergillus fumigatus* as described in PCT/US10/036461 (published as WO 2010/138754) as SEQ ID NO: 1 and SEQ ID NO: 2.

### Cellulase preparation 2:

*Trichoderma reeseicellulases, Thermoascus aurantiacus* GH61A polypeptide having cellulolytic enhancing activity (SEQ ID NO: 1 and SEQ ID NO: 2 in WO 2005/074656), *Aspergillus fumigatus* beta-glucosidase (SEQ ID NO: 2 of WO 2005/047499) and *Aspergillus aculeatus* xylanase (Xyl II disclosed in WO 94/21785).

RWB218 yeast was received from Royal Nedalco/The Netherlands and is described in Kuyper et al., 2005, FEMS Yeast Research 5: 925-934.

Unwashed pre-treated corn stover (PCS): Acid-catalyzed, steam-exploded obtained from The National Renewable Energy Laboratory (NREL), Golden, CO.

### Methods:

### Identity

The relatedness between two amino acid sequences or between two polynucleotide sequences is described by the parameter "identity".

The degree of identity between two amino acid sequences is determined by the Clustal method (Higgins, 1989, CABIOS 5: 151-153) using the LASERGENE^{™} MEGALIGN^{™} software (DNASTAR, Inc., Madison, WI) with an identity table and the following multiple alignment parameters: Gap penalty of 10 and gap length penalty of 10. Pairwise alignment parameters are Ktuple=1, gap penalty=3, windows=5, and diagonals=5.

The degree of identity between two polynucleotide sequences is determined by the Wilbur-Lipman method (Wilbur and Lipman, 1983, Proceedings of the National Academy of Science USA 80: 726-730) using the LASERGENE^{™} MEGALIGN^{™} software (DNASTAR, Inc., Madison, WI) with an identity table and the following multiple alignment parameters: Gap penalty of 10 and gap length penalty of 10. Pairwise alignment parameters are Ktuple=3, gap penalty=3, and windows=20.

### Measurement of Cellulase Activity Using Filter Paper Assay (FPU assay)

### 1. Source of Method

1.1 The method is disclosed in a document entitled "Measurement of Cellulase Activities" by Adney and Baker, 1996, Laboratory Analytical Procedure, LAP-006, National Renewable Energy Laboratory (NREL). It is based on the IUPAC method for measuring cellulase activity (Ghose, 1987, Measurement of Cellulase Activities, Pure & Appl. Chem. 59: 257-268).

### 2. Procedure

2.1 The method is carried out as described by Adney and Baker, 1996, *supra,* except for the use of a 96 well plates to read the absorbance values after color development, as described below.
*2.2 Enzyme Assay Tubes:*
   2.2.1 A rolled filter paper strip (#1 Whatman; 1 X 6 cm; 50 mg) is added to the bottom of a test tube (13 X 100 mm).
   2.2.2 To the tube is added 1.0 mL of 0.05 M Na-citrate buffer (pH 4.80).
   2.2.3 The tubes containing filter paper and buffer are incubated 5 min. at 50°C (± 0.1°C) in a circulating water bath.
   2.2.4 Following incubation, 0.5 mL of enzyme dilution in citrate buffer is added to the tube. Enzyme dilutions are designed to produce values slightly above and below the target value of 2.0 mg glucose.
   2.2.5 The tube contents are mixed by gently vortexing for 3 seconds.
   2.2.6 After vortexing, the tubes are incubated for 60 mins. at 50°C (± 0.1°C) in a circulating water bath.
   2.2.7 Immediately following the 60 min. incubation, the tubes are removed from the water bath, and 3.0 mL of DNS reagent is added to each tube to stop the reaction. The tubes are vortexed 3 seconds to mix.
*2.3 Blank and Controls*
   2.3.1 A reagent blank is prepared by adding 1.5 mL of citrate buffer to a test tube.
   2.3.2 A substrate control is prepared by placing a rolled filter paper strip into the bottom of a test tube, and adding 1.5 mL of citrate buffer.
   2.3.3 Enzyme controls are prepared for each enzyme dilution by mixing 1.0 mL of citrate buffer with 0.5 mL of the appropriate enzyme dilution.
   2.3.4 The reagent blank, substrate control, and enzyme controls are assayed in the same manner as the enzyme assay tubes, and done along with them.
2.4 *Glucose Standards*
   2.4.1 A 100 mL stock solution of glucose (10.0 mg/mL) is prepared, and 5 mL aliquots are frozen. Prior to use, aliquots are thawed and vortexed to mix.
   2.4.2 Dilutions of the stock solution are made in citrate buffer as follows:
      G1 = 1.0 mL stock + 0.5 mL buffer = 6.7 mg/mL = 3.3 mg/0.5 mL
      G2 = 0.75 mL stock + 0.75 mL buffer = 5.0 mg/mL = 2.5 mg/0.5 mL
      G3 = 0.5 mL stock + 1.0 mL buffer = 3.3 mg/mL = 1.7 mg/0.5 mL
      G4 = 0.2 mL stock + 0.8 mL buffer = 2.0 mg/mL = 1.0 mg/0.5 mL
   2.4.3 Glucose standard tubes are prepared by adding 0.5 mL of each dilution to 1.0 mL of citrate buffer.
   2.4.4 The glucose standard tubes are assayed in the same manner as the enzyme assay tubes, and done along with them.
2.5 *Color Development*
   2.5.1 Following the 60 min. incubation and addition of DNS, the tubes are all boiled together for 5 mins. in a water bath.
   2.5.2 After boiling, they are immediately cooled in an ice/water bath.
   2.5.3 When cool, the tubes are briefly vortexed, and the pulp is allowed to settle. Then each tube is diluted by adding 50 microL from the tube to 200 microL of ddH2O in a 96-well plate. Each well is mixed, and the absorbance is read at 540 nm.
*2.6 Calculations (examples are given in the NREL document)*
   2.6.1 A glucose standard curve is prepared by graphing glucose concentration (mg/0.5 mL) for the four standards (G1-G4) vs. A₅₄₀. This is fitted using a linear regression (Prism Software), and the equation for the line is used to determine the glucose produced for each of the enzyme assay tubes.
   2.6.2 A plot of glucose produced (mg/0.5 mL) vs. total enzyme dilution is prepared, with the Y-axis (enzyme dilution) being on a log scale.
   2.6.3 A line is drawn between the enzyme dilution that produced just above 2.0 mg glucose and the dilution that produced just below that. From this line, it is determined the enzyme dilution that would have produced exactly 2.0 mg of glucose.
   2.6.4 The Filter Paper Units/mL (FPU/mL) are calculated as follows:
      FPU/mL = 0.37/ enzyme dilution producing 2.0 mg glucose

### Examples

### Example 1

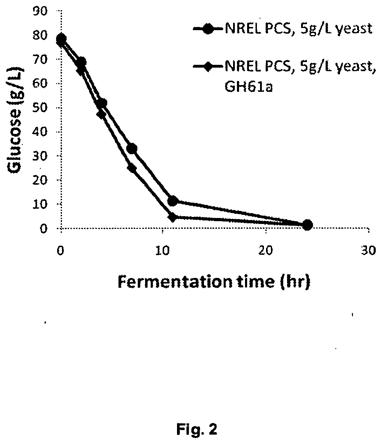

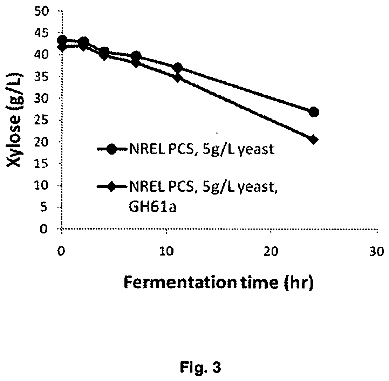

The NREL unwashed PCS was hydrolyzed at 20% total solids (TS) loading with Cellulase Preparation 2 for 8 days. Following hydrolysis, the whole slurry was centrifuged and the solid and liquid (supernatant) were separated and the supernant was fermented with 5 g/L RWB218 yeast with addition of GH61A (0.1 mg/ml). Figure 1 demonstrates the effect of the addition of GH61A on ethanol yield as measured by HPLC. Figure 2 and Figure 3 demonstrate the effect of GH61A on both glucose and xylose consumption rates.

### Example 2

NREL unwashed PCS was hydrolyzed as in Example 1. Following centrifugation, the collected supernatant was divided and treated with various GH61 polypeptides as indicated in Table 1. Table 1 demonstrates the effect of different GH61 polypeptides on ethanol yield as measured by HPLC. Values are expressed in percent ethanol yield relative to the theoretical amount of ethanol that can be produced from the substrate assuming 100% of the fermentable sugars in the substrate are converted to ethanol.

**Table 1**

| **GH61** | **TS%** | **GH61 Polypeptide Dosage (mg-Protein/g-TS)** | | | |
|---|---|---|---|---|---|
| | | 0 | 0.5 | 1 | 2 |
| *T. aurantiacus* (GH61A) | 20 | 76.7% | 81.5% | 75.7% | 83.2% |
| *A. fumigatus* (GH61B) | 16 | 79.4% | 81.4% | 81.5% | 81.2% |
| *T. terrestris* (GH61E) | 16 | 79.4% | 80.5% | 81.4% | 82.2% |

## Claims

1. A process of producing a fermentation product from lignocellulose-containing material, comprising the steps of:
(a) pre-treating lignocellulose-containing material;
(b) hydrolyzing the material of step (a);
(c) fermenting with a fermenting organism wherein one or more GH61 polypeptides are added after hydrolysis is complete.
Wherein the hydrolysis and fermentation steps are carried out as separate hydrolysis and fermentation, and the hydrolysis is taken to completion before initiation of fermentation.

2. The process of claim 1, wherein the lignocellulose-containing material originates from materials selected from the group consisting of corn stover, corn cobs, corn fiber, hardwood, softwood, cereal straw, wheat straw, switchgrass, rice hulls, Miscanthus, municipal solid waste, industrial organic waste, bagasse, and office paper, or mixtures thereof.

3. The process of any of claims 1 or 2, wherein the lignocellulose-containing material is chemically, mechanically or biologically pre-treated in step (a).

4. The process of any of claims 1-3, wherein the fermenting organism is a C6 or C5 fermenting organism.

5. The process of any of claims 1-4, wherein the fermentation product is ethanol.

6. The process of any of claims 1-5, wherein the fermentation product is recovered by distillation.

7. The process of any of claims 1-6, wherein the GH61 polypeptide is dosed at a concentration of 0.01-10 mg-protein/g total solids (TS).

8. Use of GH61 polypeptides in a fermentation process to increase the rate of fermentation during fermentation, wherein one or more GH61 polypeptides are added after hydrolysis is complete., wherein the hydrolysis and fermentation steps are carried out as separate hydrolysis and fermentation, and the hydrolysis is taken to completion before initiation of fermentation.

9. The use of claim 8, wherein the fermentation process is a process for producing ethanol.

## Patentansprüche

1. Verfahren zum Herstellen eines Fermentationsprodukts aus Lignocellulose enthaltendem Material, umfassend die Schritte:
(a) Vorbehandeln von Lignocellulose enthaltendem Material;
(b) Hydrolysieren des Materials von Schritt (a);
(c) Fermentieren mit einem fermentierenden Organismus, wobei ein oder mehrere GH61-Polypeptide hinzugefügt werden, nachdem die Hydrolyse vollständig ist,
wobei die Hydrolyse und Fermentationsschritte als separate Hydrolyse und Fermentation ausgeführt werden, und die Hydrolyse vor der Einleitung der Fermentation zur Vollständigkeit gebracht wird.

2. Verfahren nach Anspruch 1, wobei das Lignocellulose enthaltende Material aus Materialien stammt, ausgewählt aus der Gruppe bestehend aus Maisernteresten, Maiskolben, Maisfaser, Hartholz, Weichholz, Getreidestroh, Weizenstroh, Energiegras ("switchgrass"), Reisschalen, Miscanthus, festem Siedlungsabfall, gewerblichen organischen Abfällen, Bagasse und Büropapier, oder Mischungen davon.

3. Verfahren nach einem beliebigen der Ansprüche 1 oder 2, wobei das Lignocellulose enthaltende Material in Schritt (a) chemisch, mechanisch oder biologisch vorbehandelt wird.

4. Verfahren nach einem beliebigen der Ansprüche 1-3, wobei der fermentierende Organismus ein C6 oder C5 fermentierender Organismus ist.

5. Verfahren nach einem beliebigen der Ansprüche 1-4, wobei das Fermentationsprodukt Ethanol ist.

6. Verfahren nach einem beliebigen der Ansprüche 1-5, wobei das Fermentationsprodukt durch Destillation gewonnen wird.

7. Verfahren nach einem beliebigen der Ansprüche 1-6, wobei das GH61-Polypeptid in einer Konzentration von 0,01-10 mg-Protein/g Gesamtfeststoffe (*total solids,* TS) dosiert wird.

8. Verwendung von GH61-Polypeptiden in einem Fermentationsverfahren, um die Fermentationsrate während der Fermentation zu erhöhen, wobei ein oder mehrere GH61-Polypeptide hinzugefügt werden, nachdem die Hydrolyse vollständig ist, wobei die Hydrolyse und Fermentationsschritte als separate Hydrolyse und Fermentation ausgeführt werden, und die Hydrolyse vor der Einleitung der Fermentation zur Vollständigkeit gebracht wird.

9. Verwendung nach Anspruch 8, wobei das Fermentationsverfahren ein Verfahren zum Herstellen von Ethanol ist.

## Revendications

1. Procédé de production d'un produit de fermentation à partir d'un matériau contenant de la lignocellulose, comprenant les étapes de :
(a) prétraitement du matériau contenant de la lignocellulose ;
(b) hydrolyse du matériau de l'étape (a) ;
(c) fermentation avec un organisme fermenteur dans lequel un ou plusieurs polypeptides GH61 sont ajoutés après la fin de l'hydrolyse,
dans lequel les étapes d'hydrolyse et de fermentation sont réalisées sous forme d'hydrolyse et de fermentation séparées, et l'hydrolyse est menée à son terme avant le début de la fermentation.

2. Procédé selon la revendication 1, dans lequel le matériau contenant de la lignocellulose est issu de matériaux choisis dans le groupe constitué de canne de maïs, d'épis de maïs, de fibre de maïs, de bois dur, de bois tendre, de paille de céréale, de paille de blé, de panic raide, d'écorce de riz, de Miscanthus, de déchets municipaux solides, de déchets organiques industriels, de bagasse, et de papier de bureau, ou des mélanges de ceux-ci.

3. Procédé selon l'une quelconque des revendications 1 ou 2, dans lequel le matériau contenant de la lignocellulose est prétraité chimiquement, mécaniquement ou biologiquement durant l'étape (a).

4. Procédé selon l'une quelconque des revendications 1 à 3, dans lequel l'organisme fermenteur est un organisme fermenteur en C6 ou C5.

5. Procédé selon l'une quelconque des revendications 1 à 4, dans lequel le produit de fermentation est l'éthanol.

6. Procédé selon l'une quelconque des revendications 1 à 5, dans lequel le produit de fermentation est récupéré par distillation.

7. Procédé selon l'une quelconque des revendications 1 à 6, dans lequel le polypeptide GH61 est dosé à une concentration de 0,01 à 10 mg de protéine/g de matières solides totales (ST).

8. Utilisation de polypeptides GH61 dans un procédé de fermentation pour augmenter le taux de fermentation pendant la fermentation, dans lequel un ou plusieurs polypeptides GH61 sont ajoutés après le fin de l'hydrolyse, dans laquelle les étapes d'hydrolyse et de fermentation sont réalisées sous forme d'hydrolyse et de fermentation séparées, et l'hydrolyse est menée à son terme avant le début de la fermentation.

9. Utilisation selon la revendication 8, dans laquelle le procédé de fermentation est un procédé pour la production d'éthanol.
